(19) Europäisches Patentamt
European Patent Office
Office européen des brevets

(11) Veröffentlichungsnummer: **0 039 771**
**B1**

(12) # EUROPÄISCHE PATENTSCHRIFT

(45) Veröffentlichungstag der Patentschrift: **19.10.83**

(21) Anmeldenummer: **81102377.9**

(22) Anmeldetag: **30.03.81**

(51) Int. Cl.³: **C 07 C 93/06**, C 07 C 121/75, C 07 C 149/31, C 07 C 89/00, A 61 K 31/135

(54) **Cyclopropanderivate, diese enthaltende pharmazeutische Zubereitungen und Verfahren zu ihrer Herstellung.**

(30) Priorität: **09.05.80 DE 3017812**

(43) Veröffentlichungstag der Anmeldung: **18.11.81 Patentblatt 81/46**

(45) Bekanntmachung des Hinweises auf die Patenterteilung: **19.10.83 Patentblatt 83/42**

(84) Benannte Vertragsstaaten: **AT BE CH DE FR GB IT LI NL SE**

(56) Entgegenhaltungen:
**CH-A-485 645**
**DD-A-118 613**
**GB-A-951 461**

(73) Patentinhaber: **Merck Patent Gesellschaft mit beschränkter Haftung, Frankfurter Strasse 250, D-6100 Darmstadt (DE)**

(72) Erfinder: **Hausberg, Hans-Heinrich, Dr., Odenwaldstrasse 30, D-6105 Ober-Ramstadt (DE)**
Erfinder: **Uhl, Jürgen, Dr., Odenwaldstrasse 49, D-6104 Seeheim 1 (DE)**
Erfinder: **Seyfried, Christoph, Dr., Mathildenstrasse 6, D-6104 Seeheim-Jugenheim 2 (DE)**
Erfinder: **Minck, Klaus-Otto, Dr., Büchestrasse 8, D-6105 Ober-Ramstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 039 771 B1

BUNDESDRUCKEREI BERLIN

Cyclopropanderivate, diese enthaltende pharmazeutische Zubereitungen und Verfahren zu ihrer Herstellung

Die Erfindung betrifft neue Cyclopropanderivate der allgemeinen Formel I

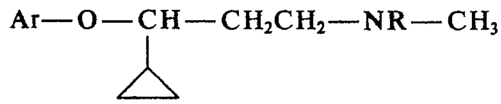

I

worin

Ar Phenyl oder eine ein- oder mehrfach durch F, Cl, Br, Alkyl, Alkoxy, Alkenyloxy und/oder Alkylthio mit jeweils bis zu 4 C-Atomen, OH, SH, CN, Methylendioxy und/oder $CF_3$ substituierte Phenylgruppe und

R H, Alkyl mit 1–4 C-Atomen oder Benzyl

bedeuten, sowie ihre physiologisch unbedenklichen Säureadditionssalze.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem antidepressive Wirkungen. Im einzelnen kann eine reserpinantagonistische Wirkung (feststellbar z. B. an Mäusen gegenüber Reserpin in Anlehnung an die Methode von Askew, Life Science, Bd. 10 [1963], Seiten 725–730), eine antikataleptische Wirkung (feststellbar z. B. an Ratten gegenüber Tetrabenazin in Anlehnung an die Methode von Giurgea et al., Medicina Experimentalis, Bd. 9 [1963], Seiten 249–262) und eine antiptotische Wirkung (feststellbar z. B. gegenüber Reserpin in Anlehnung an die Methodik von Domenjoz und Theobald, Arch. int. pharmacodyn., Bd. 120 [1959], Seite 450 ff., mit der Bewertung nach Rubin et al., J. Pharmacol. Exp. Therap., Bd. 120 [1957], Seiten 125–136) nachgewiesen werden. Weiterhin kann die Wirkung des 5-Hydroxytryptophans bei Mäusen (Methode: ähnlich wie Ross et al., Acta pharmacol. et toxicol., Bd. 39 [1976], Seiten 152–166) potenziert werden und die zentralen Auswirkungen einer Erregung und Temperatursteigerung, die D-Amphetaminsulfat (z. B. 1,5 mg/kg s.c., 1 Stunde nach der Prüfsubstanz verabfolgt, die ebenfalls s.c. appliziert wird) und Aggregation (Zusammensetzung von 5 Ratten in einem Glas) auslösen (Methodik nach Müller–Calgan et al. in Zippel, H. P. [Ed.]: Memory and Transfer of Information, Plenum Press, New York–London, 1973, Seiten 87–125) können, erhöht und/oder verlängert werden. Die Substanzen haben einen Einfluß auf die biogenen Amine des ZNS. So führen sie z. B. in vitro zur Aufnahmehemmung von Noradrenalin, 5-Hydroxytryptamin und Dopamin (Methodik: Kannengießer et al., Biochem. Pharmacol., Bd. 22 [1973], Seiten 73–84) in Synaptosomen und hemmen in vivo (Methodik: Carlsson et al., Europ. J. Pharmacol., Bd. 5 [1969], Seiten 357–366; 367–373) die durch Tyraminderivate induzierte Katecholamin- und Serotonin-Freisetzung im Gehirn. Ferner antagonisieren die Verbindungen die durch p-Chloramphetamin hervorgerufene Serotoninspiegelsenkung bei Ratten bei oraler Applikation (Methodik: Fuller et al., Biochem. Pharmacol., Bd. 27 [1978], 193–198). Weiterhin treten blutdrucksenkende und spasmolytische Wirkungen auf, die nach hierfür geläufigen Methoden ermittelt werden können.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Cyclopropanderivate der Formel I sowie ihre physiologisch unbedenklichen Säureadditionssalze.

In den Resten Ar und R bedeutet Alkyl vorzugsweise Methyl, ferner auch Äthyl, n-Propyl, Isopropyl, n-Butyl, Isobutyl, sek.-Butyl oder tert.-Butyl. Alkoxy (im Rest Ar) ist vorzugsweise Methoxy, ferner auch Äthoxy, n-Propoxy, Isopropoxy, n-Butoxy, Isobutoxy, sek.-Butoxy oder tert.-Butoxy. Alkenyloxy (im Rest Ar) ist vorzugsweise Allyloxy, ferner auch Vinyloxy, Propenyloxy, Isopropenyloxy, 1-Buten-1- oder -2-yloxy, 2-Buten-1- oder -2-yloxy, 3-Buten-1- oder -2-yloxy, 2-Methyl-1-propen-1- oder -2-yloxy oder 2-Methyl-2-propen-1-yloxy.

Alkylthio (im Rest Ar) steht vorzugsweise für Methylthio, ferner auch für Äthylthio, n-Propylthio, Isopropylthio, n-Butylthio, Isobutylthio, sek.-Butylthio oder tert.-Butylthio.

Der Rest Ar ist vorzugsweise eine einfach substituierte Phenylgruppe, er kann aber auch insbesondere für eine unsubstituierte oder eine zweifach substituierte Phenylgruppe stehen, ferner auch für eine dreifach, vierfach oder fünffach substituierte Phenylgruppe.

Im einzelnen bedeutet der Rest Ar vorzugsweise Fluorphenyl, Chlorphenyl, Methoxyphenyl, Allyloxyphenyl, Methylthiophenyl, Hydroxyphenyl, Mercaptophenyl, Cyanphenyl oder insbesondere Trifluormethylphenyl, im einzelnen insbesondere m- oder p-Fluorphenyl, p-Chlorphenyl, o-, m- oder p-Methoxyphenyl, o-Allyloxyphenyl, p-Methylthiophenyl oder p-Trifluormethylphenyl. Weiterhin

steht Ar vorzugsweise für o-, m- oder p-Tolyl, o-, m- oder p-Äthylphenyl, o-Fluorphenyl, o- oder m-Chlorphenyl, o-, m- oder p-Bromphenyl, o-, m- oder p-Äthoxyphenyl, o-, m- oder p-n-Propoxyphenyl, o-, m- oder p-Isopropoxyphenyl, o-, m- oder p-Vinyloxyphenyl, m- oder p-Allyloxyphenyl, o- oder m-Methylthiophenyl, o-, m- oder Äthylthiophenyl, o-, m- oder p-Hydroxyphenyl, o-, m- oder p-Mercaptophenyl, o-, m- oder p-Cyanphenyl, 3,4-Methylendioxyphenyl, o- oder m-Trifluormethylphenyl. Unter den zweifach substituierten Phenylgruppen ist Dimethoxyphenyl bevorzugt, insbesondere 3,4-Dimethoxyphenyl, unter den dreifach substituierten Trimethoxyphenyl, insbesondere 3,4,5-Trimethoxyphenyl.

Der Rest R steht vorzugsweise für H, ferner vorzugsweise für Methyl oder Benzyl, weiterhin insbesondere für Äthyl.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ie ausgedrückt werden, die der Formel I entsprechen und worin die nicht näher bezeichneten Reste die bei Formel I angegebene Bedeutung haben, worin jedoch

in Ia Ar Phenyl oder eine einfach durch F, Cl, $CH_3O$, $CH_2=CHCH_2O$, $CH_3S$, OH, SH, CN oder $CF_3$ substituierte Phenylgruppe und
R H, $CH_3$ oder Benzyl bedeuten;

in Ib Ar m- oder p-Fluorphenyl, p-Chlorphenyl, o-, m- oder p-Methoxyphenyl, o-Allyloxyphenyl, p-Methylthiophenyl oder p-Trifluormethylphenyl und
R H, $CH_3$ oder Benzyl bedeuten;

in Ic Ar m- oder p-Fluorphenyl, p-Chlorphenyl, o-, m- oder p-Methoxyphenyl, o-Allyloxyphenyl, p-Methylthiophenyl oder p-Trifluormethylphenyl und
R H oder $CH_3$ bedeuten;

in Id Ar o-Methoxyphenyl oder p-Trifluormethylphenyl und
R H, $CH_3$ oder Benzyl bedeuten;

in Ie Ar p-Trifluormethylphenyl und
R H, $CH_3$ oder Benzyl bedeuten.

Die Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrerer Racemate sowie in verschiedenen optisch-aktiven Formen vorliegen.

Gegenstand der Erfindung ist ferner ein Verfahren zur Herstellung der Verbindungen der Formel I sowie ihrer physiologisch unbedenklichen Säureadditionssalze, dadurch gekennzeichnet, daß man ein Phenol der allgemeinen Formel II

$$Ar-OH \qquad II$$

worin

Ar die angegebene Bedeutung hat,

oder eines seiner Salze mit einem Amin der allgemeinen Formel III

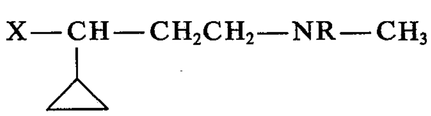

III

worin

X Cl, Br, J oder OH bedeutet und
R die angegebene Bedeutung hat,

oder mit einem seiner reaktionsfähigen Derivate umsetzt

oder daß man ein Cyclopropanderivat der allgemeinen Formel IV

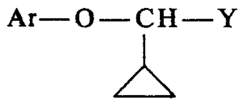

IV

worin

Y  $-CH_2CH_2X$ oder $-CH=CH_2$ bedeutet und
Ar und X  die angegebenen Bedeutungen haben,

oder eines seiner reaktionsfähigen Derivate mit einem Amin der allgemeinen Formel V

$HNR-CH_3$  V

worin

R  die angegebene Bedeutung hat,

umsetzt

oder daß man ein Amin der allgemeinen Formel VI

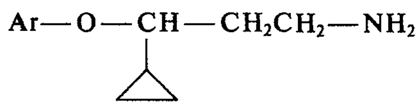

VI

worin

Ar  die angegebene Bedeutung hat,

mit einem alkylierenden Mittel behandelt
oder daß man eine Verbindung, die der allgemeinen Formel I entspricht, jedoch an Stelle eines Wasserstoffatoms zusätzlich eine solvolytisch abspaltbare Gruppe enthält,

mit einem solvolysierenden Mittel behandelt
oder daß man eine Verbindung, die der allgemeinen Formel I entspricht, jedoch an Stelle von Wasserstoffatomen eine oder mehrere reduzierbare Gruppen und/oder eine oder mehrere C–C- und/oder C–N-Mehrfachbindungen enthält, mit einem reduzierenden Mittel behandelt und/oder daß man gegebenenfalls in einer Verbindung der Formel I eine sekundäre Aminogruppe durch Behandeln mit einem lakylierenden oder benzylierenden Mittel in die entsprechende tertiäre Aminogruppe und/oder eine Hydroxygruppe durch Behandeln mit einem alkylierenden oder alkenylierenden Mittel in die entsprechende Alkoxy- oder Alkenyloxygruppe und/oder eine Mercaptogruppe durch Behandeln mit einem alkylierenden Mittel in die entsprechende Alkylthiogruppe und/oder eine N-Benzylgruppe durch Behandeln mit einem entbenzylierenden Mittel (falls erwünscht, stufenweise) in eine NH-Gruppe umwandelt und/oder eine Alkoxy- und/oder Alkylthiogruppe unter Bildung einer Hydroxy- und/oder Mercaptogruppe spaltet und/oder eine erhaltene Base der Formel I durch Behandeln mit einer Säure in eines ihrer physiologisch unbedenklichen Säureadditionssalze umwandelt.

Die Herstellung der Verbindungen der Formel I erfolgt im übrigen nach an sich bekannten Methoden, wie sie in der Literatur (z. B. in den Standardwerken wie Houben–Weyl, Methoden der Organischen Chemie, Georg Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formeln II und III können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I werden vorzugsweise durch Reaktion der Phenole der Formel II oder — bevorzugt — ihrer Salze mit den Aminen der Formel III erhalten.

Die Phenole der Formel II sind größtenteils bekannt und können nach an sich bekannten Methoden hergestellt werden, z. B. durch Spaltung entsprechender Benzyl- oder Methyläther.

In den Verbindungen der Formeln III und IV bedeutet der Rest X vorzugsweise Cl oder Br. Reaktionsfähige Derivate dieser Verbindungen sind insbesondere die Reaktionsfähigen Ester der

Alkohole der Formeln III und IV (X = OH), vorzugsweise die entsprechenden Alkylsulfonate (worin die Alkylgruppe 1 – 6 C-Atome besitzt) und die entsprechenden Arylsulfonate (worin die Arylgruppe 6 – 10 C-Atome besitzt), z. B. die entsprechenden Methan-, Benzol-, p-Toluol- oder Naphthalin-1- oder -2-sulfonate.

Die Basen der Formel III können nach an sich bekannten Methoden hergestellt werden. So sind die Alkohole der Formel III (X = OH) z. B. erhältlich durch Mannich-Reaktion aus Cyclopropylmethylketon, Formaldehyd und Aminen der allgemeinen Formel $HNR-CH_3$ zu Ketonen der allgemeinen Formel Cyclopropyl – $COCH_2CH_2-NR-CH_3$ und anschließende Reduktion dieser Ketone, die Verbindungen der Formel III (X = Cl, Br oder J) aus den Alkoholen mit anorganischen Halogeniden wie $SOCl_2$, $PBr_3$ oder HJ, die Sulfonate durch Veresterung der Alkohole mit den entsprechenden Sulfonylchloriden. Die tertiären unter den Aminen der Formel III (in denen R nicht H bedeutet) sind auch aus den sekundären Aminen (III, R = H) durch Alkylierung oder Benzylierung zugänglich. Umgekehrt können die sekundären Amine (III, R = H) aus den entsprechenden N-Alkyl- oder N-Benzylderivaten (III, R = Alkyl mit 1 – 4 C-Atomen) durch Desalkylierung oder Desbenzylierung mit Chlorameisensäure-äthyl- oder -trichloräthylester erhalten werden.

Vor der Umsetzung mit dem Amin III wird das Phenol II zweckmäßig zunächst in ein Salz übergeführt, insbesondere in ein Metallsalz, z. B. ein Alkalimetallsalz (Li-, Na- oder K-Salz) oder Thaliumsalz. Man kann II mit einem metallsalz-bildenden Reagenz umsetzen, z. B. einem Alkalimetall (z. B. Na), einem Alkalimetallhydrid oder -amid (z. B. LiH, NaH, $NaNH_2$ oder $KNH_2$), einem Metallalkoholat (worin der Alkohol-Teil vorzugsweise 1 – 4 C-Atome besitzt, z. B. Lithium-, Natrium-, Kalium- oder Thalliummethylat, -äthylat oder -tert.-butylat), einer Organometallverbindung (z. B. Butyllithium, Phenyllithium oder Phenylnatrium), einem Metallhydroxid, -carbonat oder -bicarbonat (z. B. des Li, Na, K oder Ca). Die Herstellung des Salzes wird vorteilhafterweise in Gegenwart eines Lösungsmittels oder Lösungsmittelgemisches vorgenommen. Geeignete Lösungsmittel sind z. B. Kohlenwasserstoffe (wie Hexan, Benzol, Toluol oder Xylol), Äther (wie Diäthyläther, Diisopropyläther, Tetrahydrofuran [THF], Dioxan oder Diäthylenglykoldimethyläther), Amide wie Dimethylformamid (DMF), Alkohole (wie Methanol oder Äthanol).

Das Phenol II oder dessen Salz wird mit dem Amin III vorzugsweise in Gegenwart eines Verdünnungsmittels umgesetzt, z. B. desjenigen Lösungsmittels, das für die Herstellung des Salzes verwendet worden ist, das jedoch durch ein anderes Lösungsmittel ersetzt oder mit einem solchen verdünnt sein kann.

Die Umsetzung wird in der Regel bei Temperaturen zwischen etwa – 20 und 150° durchgeführt, vorzugsweise zwischen 20 und 120°.

Das Phenolat kann auch in situ gebildet werden. In diesem Fall läßt man das Phenol II und das Amin III miteinander in Gegenwart einer Base reagieren.

Eine Variante der Reaktion besteht darin, daß man das Phenol der Formel II mit einem Hydroxyamin der Formel III (X = OH), in Gegenwart eines Dehydratisierungsmittels, z. B. eines Azodicarbonsäuredialkylesters in Anwesenheit von Triphenylphosphin in einem inerten Lösungsmittel wie THF bei etwa – 10 bis + 30° umsetzt.

Die Cyclopropanderivate der Formel I sind weiterhin durch Reaktion von Verbindungen der Formel IV mit Aminen der Formel V erhältlich.

Die Ausgangsstoffe der Formel IV ($Y = -CH_2CH_2OH$) sind beispielsweise erhältlich durch Umsetzung von Cyclopropylcyanid mit Organometallverbindungen der Formel $M-CH_2CH(OAlkyl)_2$ (worin M Li, MgCl oder MgBr bedeutet) und anschließende Hydrolyse zu 3,3-Dialkoxy-1-cyclopropyl-propan-1-onen, Reduktion zu 3,3-Dialkoxy-1-cyclopropyl-propan-1-olen, Verätherung mit Phenolen der Formel Ar – OH zu den entsprechenden 3,3-Dialkoxy-1-cyclopropyl-1-aryloxy-propanen, Hydrolyse zu 3-Cyclopropyl-3-aryloxy-propanalen und Reduktion. Verbindungen der Formel IV ($Y = -CH=CH_2$) sind daraus durch Dehydratisierung erhältlich, Verbindungen der Formel IV ($Y = -CH_2CH_2Cl$, $-CH_2CH_2Br$ oder $-CH_2CH_2J$) durch Reaktion mit anorganischen Halogeniden wie $SOCl_2$, $PBr_3$ oder HJ, die entsprechenden Sulfonate durch Veresterung der Alkohole (IV, $Y = CH_2CH_2OH$) mit Sulfonylchloriden.

Die Umsetzung der Verbindungen der Formeln IV und V erfolgt unter Alkylierungsbedingungen, zweckmäßig in Gegenwart eines der genannten inerten Lösungsmittel bei Temperaturen zwischen etwa 20 und 140°, vorzugsweise 80 und 120°. Verwendet man leicht flüchtige Ausgangsstoffe der Formel V, so kann es vorteilhaft sein, unter Druck (bis zu etwa 100 at) zu arbeiten.

Die Cyclopropanderivate der Formel I können auch hergestellt werden aus Aminen der Formel VI durch Alkylierung, insbesondere Methylierung.

Die Amine der Formel VI können z. B. aus den Verbindungen der Formel IV und Ammoniak hergestellt werden.

Alkylierung der Amine der Formel VI erfolgt zweckmäßig unter gleichen Bedingungen wie die Reaktion von IV mit V.

Man kann die Amine der Formel VI mit Aldehyden oder Ketonen unter Bildung von Aldehyd-Ammoniak-Verbindungen, Schiffschen Basen oder Enaminen kondensieren und diese anschließend entweder hydrieren oder mit einem Alkylierungsmittel behandeln und das erhaltene quartäre Salz anschließend hydrolysieren. Beispielsweise kann man ein Amin der Formel VI durch

Kondensation mit Benzaldehyd in die N-Benzylidenverbindung überführen und diese mit einem Methylhalogenid in eines ihrer quartären Salze umwandeln, das nachfolgend z. B. durch Behandeln mit wässerigem Alkohol unter Abspaltung von Benzaldehyd in ein sekundäres Amin der Formel I (R = H) übergeführt werden kann. Man kann ferner mit Aldehyden oder Ketonen unter reduzierenden Bedingungen alkylieren, wobei als Zwischenprodukte die entsprechenden Aldehyd-Ammoniake entstehen. Beispielsweise kann man eine oder zwei Methylgruppen mit Formaldehyd in Gegenwart von Ameisensäure einführen. Weiterhin kann mit einem Alkohol in Gegenwart von Raney-Nickel alkylieren.

Ferner kann man in einer Verbindung, die der Formel I entspricht, jedoch an Stelle eines Wasserstoffatoms zusätzlich eine solvolytisch abspaltbare Gruppe (vorzugsweise eine N-Acyl-, O-Acyl-, S-Acyl- oder N-Cyangruppe) enthält, diese Gruppe durch Behandeln mit einem solvolysierenden Mittel abspalten.

Bevorzugte Ausgangsstoffe für diese Solvolyse sind solche der allgemeinen Formel VII

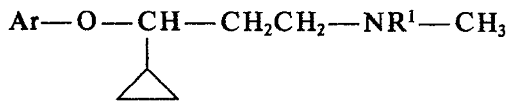

VII

worin

$R^1$ CN oder eine Acylgruppe mit 1–7 C-Atomen, vorzugsweise Acetyl, Trifluoracetyl oder Benzoyl bedeutet und

Ar die angegebene Bedeutung hat.

Die Ausgangsstoffe der Formel VII sind z. B. erhältlich durch Reaktion von Verbindungen der Formel IV mit Verbindungen der Formel $HNR^1-CH_3$ oder durch Reaktion von Verbindungen der Formel Cyclopropyl $-CHX-CH_2-CH_2-NR^1-CH_3$ mit Phenolen der Formel Ar $-$ OH (II).

Ferner sind Ausgangsstoffe geeignet, die der Formel I entsprechen, aber im Rest Ar an Stelle einer OH- oder SH-Gruppe eine Acyloxy- oder Acylthiogruppe mit jeweils 1–7 C-Atomen im Acylrest tragen.

Die Solvolyse dieser Verbindungen gelingt zweckmäßig durch Einwirkung eines Lösungsmittels wie Wasser (Hydrolyse) oder eines Alkohols mit vorzugsweise 1–4 C-Atomen (Alkoholyse) in Gegenwart eines sauren oder basischen Katalysators, z. B. einer Mineralsäure wie Schwefelsäure oder Salzsäure, eines Metallhydroxids wie Natrium-, Kalium-, Calcium-, Barium-, Blei- oder Silberhydroxid, oder eines Metall- oder Ammoniumsalzes wie Natrium- oder Kaliumcarbonat oder Ammoniumchlorid. Als Alkohole dienen vorzugsweise Methanol, Äthanol oder Isopropanol, man kann auch Gemische von Wasser mit einem dieser Alkohole verwenden. Die Solvolyse erfolgt zweckmäßig bei Temperaturen zwischen etwa 0 und etwa 120°.

Im einzelnen hydrolysiert man zweckmäßig durch 1–24stündiges Kochen mit wässeriger, wässerig-alkoholischer, wässerig-äthylenglykolischer oder alkoholischer Natronlauge oder Kalilauge.

Die Cyclopropanderivate der Formel I können ferner hergestellt werden durch Reduktion entsprechender Ausgangsstoffe, die zusätzlich reduzierbare Gruppen und/oder C–C- und/oder C–N-Doppel- oder -Dreifachbindungen enthalten.

Unter den reduzierbaren Ausgangsstoffen sind solche der allgemeinen Formel VIII bevorzugt.

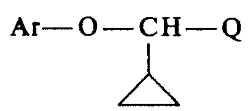

VIII

worin

Q $-CH=CH-NR-CH_3$, $-CH_2CH=N-R$, $-CH_2-CH_2-N=CH_2$, $-CO-CH_2-NR-CH_3$, $-CH_2-CO-NR-CH_3$ oder $-CH_2-CH_2-NR^2-CH_3$ und

$R^2$ eine hydrogenolytisch abspaltbare Aminoschutzgruppe oder eine Hydroxyalkyl-, Oxoalkyl-, Alkenyl- oder Alkinylgruppe mit jeweils bis zu 4 C-Atomen bedeutet und

Ar und R die angegebenen Bedeutungen haben.

Die Verbindungen der Formel VIII können beispielsweise erhalten werden durch Umsetzung von Phenolen der Formel Ar $-$ OH (II) mit Cyclopropanderivaten der Formel Cyclopropyl $-CHX-Q$ (worin Ar, X und Q die angegebenen Bedeutungen haben). Schiffsche Basen der Formel VIII, worin Q $-CH_2-CH=N-R$ bedeutet, können auch aus den obengenannten 3-Cyclopropyl-3-aryloxypropanalen und Ammoniak bzw. Aminen der Formel $R-NH_2$ erhalten werden. Amide der Formel VIII, worin Q $-CH_2-CO-NR-CH_3$ bedeutet, auch durch Amidierung entsprechender 3-Cyclopropyl-3-aryloxypropansäuren oder ihrer Ester.

Ferner sind z. B. Ausgangsstoffe geeignet, die der Formel I entsprechen, aber im Rest Ar an Stelle

einer OH- oder SH-Gruppe eine Benzyloxy- oder Benzylthiogruppe tragen.

Die Ausgangsstoffe der Formel VIII und die übrigen reduzierbaren Ausgangsstoffe können beispielsweise durch katalytische Hydrierung, mit nascierendem Wasserstoff, mit komplexen Metallhydriden oder mit Hilfe anderer chemischer Reduktionsmittel in die Verbindungen der Formel I umgewandelt werden. Die für die einzelnen Ausgangsstoffe geeigneten Reduktionsmethoden sind im allgemeinen von der Art der Gruppe abhängig und dem Fachmann nach den Angaben der Literatur geläufig. So können z. B. Schiffsche Basen und olefinische Verbindungen besonders vorteilhaft katalytisch hydriert werden. Eine Reduktion der Säureamide erfolgt dagegen besonders vorteilhaft mit komplexen Metallhydriden oder mit Diboran.

Für katalytische Hydrierungen eignen sich beispielsweise Edelmetall-, Nickel- oder Kobaltkatalysatoren, ferner auch Mischkatalysatoren wie Kupferchromoxid. Als Edelmetalle kommen in erster Linie Platin und Palladium in Betracht, die auf Trägern (z. B. auf Kohle, Calciumcarbonat oder Strontiumcarbonat), als Oxide (z. B. Platinoxid) oder in feinteiliger Form vorliegen können. Nickel- und Kobaltkatalysatoren werden zweckmäßig als Raney-Metalle eingesetzt. Man kann zweckmäßig bei Drücken zwischen etwa 1 und 200 at und bei Temperaturen zwischen etwa $-80$ und $+150°$ hydrieren. Die Hydrierung erfolgt in Gegenwart eines inerten Lösungsmittels, z. B. eines Alkohols wie Methanol, Äthanol oder Isopropanol, einer Carbonsäure wie Essigsäure, eines Esters wie Äthylacetat, eines Äthers wie Tetrahydrofuran (THF) oder Dioxan. Man kann auch Lösungsmittelgemische verwenden, z. B. auch Wasser enthaltende Gemische. Bevorzugt ist eine Hydrierung unter milden Bedingungen, z. B. bei Temperaturen zwischen 0 und 30° unter Normaldruck.

Ferner können als Reduktionsmittel komplexe Metallhydride wie $LiAlH_4$, $NaBH_4$ oder $NaAl(OCH_2CH_2OCH_3)_2H_2$ sowie Diboran eingesetzt werden, falls erwünscht, unter Zusatz von Katalysatoren wie $BF_3$, $AlCl_3$ oder LiBr. Als Lösungsmittel eignen sich hierfür insbesondere Äther wie Diäthyläther, THF, Dioxan, 1,2-Dimethoxyäthan oder Diglyme sowie Kohlenwasserstoffe wie Benzol. Für eine Reduktion mit $NaBH_4$ sind in erster Linie Alkohole wie Methanol oder Äthanol als Lösungsmittel geeignet. Nach dieser Methode reduziert man vorzugsweise bei Temperaturen zwischen etwa $-80$ und $+150°$, insbesondere zwischen etwa 20 und 120°.

Weiterhin ist als Reduktionsmethode die Umsetzung mit nascierendem Wasserstoff geeignet. Dieser kann beispielsweise durch Behandlung von Metallen mit Säuren oder Basen erzeugt werden. So können z. B. die Systeme Zink/Säure, Zink/Alkalilauge, Eisen/Säure oder Zinn/Säure verwendet werden. Als Säuren eignen sich z. B. Salzsäure oder Essigsäure. Auch ein Alkalimetall wie Natrium in einem Alkohol wie Äthanol, Isopropanol, n-Butanol, Amylalkohol, Isoamylalkohol oder in Phenol kann als Reduktionsmittel verwendet werden, ferner z. B. eine Aluminium-Nickel-Legierung in alkalisch-wässeriger oder alkalisch-wässerig-alkoholischer Lösung, sowie Natrium- oder Aluminiumamalgam in wässerig-alkoholischer oder wässeriger Lösung. Bei diesen Methoden liegen die Reaktionstemperaturen zwischen etwa 0 und etwa 150°, vorzugsweise zwischen etwa 20 und 120°.

Weiterhin kann man, falls erwünscht, sekundäre Amine der Formel I (R = H) am Stickstoffatom alkylieren, wobei man tertiäre Amine der Formel I (R = Alkyl mit 1–4 C-Atomen) erhält. Als N-Alkylierungsmittel eignen sich z. B. die entsprechenden Alkylhalogenide, z. B. Methylchlorid, Methylbromid, Methyljodid, Äthylchlorid, Äthylbromid, Äthyljodid, n-Propylchlorid, -bromid oder -jodid usw., ferner die entsprechenden Dialkylsulfate wie Dimethylsulfat und die entsprechenden Sulfonsäurealkylester wie p-Toluolsulfonsäure-methylester. Eine Methylgruppe kann ferner beispielsweise durch Behandeln mit Ameisensäure und wässeriger Formaldehydlösung eingeführt werden, zweckmäßig durch mehrstündiges Erhitzen auf Temperaturen zwischen 50 und 100°. Allgemein wird die N-Alkylierung zweckmäßig in Gegenwart oder Abwesenheit eines inerten Lösungsmittels bei Temperaturen zwischen etwa 0 und etwa 120°, vorzugsweise zwischen 40 und 100°, vorgenommen, wobei auch ein Katalysator zugegen sein kann, vorzugsweise eine Base wie Kalium-tert.-butylat.

Eine Alkylierung gelingt auch durch Behandeln eines sekundären Amins I (R = H) mit einem Aldehyd oder Keton in Gegenwart von Wasserstoff und einem Hydrierungskatalysator (z. B. Raney-Nickel) bei Temperaturen zwischen etwa 50 und 100° und Drücken zwischen etwa 1 und 200 at; so erhält man mit Aceton die entsprechende Isopropylverbindung I (R = Isopropyl).

Eine Alkylierung ist auch mehrstufig möglich. Man kann beispielsweise ein Amin der Formel I (R = H) zunächst in an sich bekannter Weise acylieren (z. B. durch Behandeln mit Acetanhydrid/Pyridin acetylieren) und das erhaltene N-Acylierungsprodukt (z. B. N-Acetylierungsprodukt) anschließend zum gewünschten tertiären Amin reduzieren, beispielsweise mit einem komplexen Metallhydrid wie $LiAlH_4$ in einem inerten Lösungsmittel wie Diäthyläther oder THF, vorzugsweise bei Temperaturen zwischen 20 und 60°.

In ganz analoger Weise kann ein sekundäres Amin der Formel I (R = H) mit benzylierenden Mitteln (z. B. Benzylhalogeniden wie Benzylchlorid oder -bromid) behandelt werden, wobei tertiäre Amine der Formel I (R = Benzyl) gebildet werden.

In ähnlicher Weise kann ein Phenol der Formel I (Ar = eine durch eine oder mehrere OH-Gruppen substituierte Phenylgruppe) alkyliert oder alkenyliert werden, wobei man eine Verbindung der Formel I (Ar = eine durch eine oder mehrere Alkoxy- und/oder Alkenyloxygruppen substituierte Phenylgruppe) erhält. Als Alkylierungsmittel eignen sich z. B. die obenangegebenen Alkylhalogenide, Dialkylsulfate und Sulfonsäurealkylester, als Alkenylierungsmittel die entsprechenden Alkenylhalogenide (z. B.

Allylchlorid, -bromid oder -jodid).

Die O-Alkylierung (oder Alkenylierung) wird zweckmäßig bei Temperaturen zwischen etwa 0 und etwa 150°, vorzugsweise zwischen 20 und 100°, in einem inerten Lösungsmittel vorgenommen, z. B. einem Alkohol wie Methanol oder Äthanol, einem Kohlenwasserstoff wie Benzol, einem Amid wie DMF, einem Äther wie THF oder einem Amin wie Pyridin. Verwendet man Halogenide, so arbeitet man zweckmäßig in Gegenwart einer Base wie NaOH, KOH, Triäthylamin oder Pyridin, wobei ein Überschuß dieser Base als Lösungsmittel dienen kann.

In ganz analoger Weise ist eine S-Alkylierung von Mercaptanen der Formel I (Ar = eine durch eine oder mehrere SH-Gruppen substituierte Phenylgruppe) möglich, wobei man die entsprechenden Alkylthioverbindungen erhält.

Weiterhin kann in einer Verbindung der Formel I (R = Benzyl) die Benzylgruppe nach an sich bekannten Methoden entfernt werden, z. B. durch Hydrogenolyse in Gegenwart eines Edelmetallkatalysators oder stufenweise mit Hilfe eines desalkylierenden Agens, z. B. durch Reaktion mit Chloramiesensäure-äthyl-, -phenyl-, -trichlormethyl- oder -2,2,2-trichloräthylester in Gegenwart einer Base zum entsprechenden Urethan, das anschließend reduktiv, z. B. mit Zink/Essigsäure, oder hydrolytisch, z. B. mit einer Base, gespalten wird.

Äther der Formel I (Ar = eine durch eine oder mehrere Alkoxygruppen substituierte Phenylgruppe) können nach Methoden, die aus der Literatur bekannt sind, gespalten werden. Dabei entstehen die entsprechenden Phenole der Formel I (Ar = eine durch eine oder mehrere OH-Gruppen substituierte Phenylgruppe). Zum Beispiel kann man die Ätherspalten durch Behandeln mit HBr oder HJ in wässeriger oder essigsaurer Lösung, durch Erhitzen mit Lewis-Säuren wie $AlCl_3$ oder Bortrihalogeniden oder durch Verschmelzen mit Pyridin- oder Anilin-hydrohalogeniden, vorzugsweise Pyridinhydrochlorid, bei etwa 150 – 250°.

Analog können thioäther der Formel I (Ar = eine durch eine oder mehrere Alkylthiogruppen substituierte Phenylgruppe) zu den entsprechenden Mercaptoverbindungen der Formel I (Ar = eine durch eine oder mehrere Mercaptogruppen substituierte Phenylgruppe) gespalten werden.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diäthylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Äthansulfonsäure, Äthandisulfonsäure, 2-Hydroxyäthansulfonsäure, Benzolsulfonsäure, p-Toluolsulfonsäure, Naphthalin-mono- und -disulfonsäuren, Laurylschwefelsäure. Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Weg. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend eine Verbindung der Formel I und/oder eines ihrer physiologisch unbedenklichen Säureadditionssalze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyäthylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Gegenstand der Erfindung ist ferner die Verwendung der Verbindungen der Formel I und ihrer physiologisch unbedenklichen Säureadditionssalze bei der Bekämpfung von Krankheiten, insbesondere von Depressionen verschiedener Äthiologie und Symptomatologie, sowie ihre Verwendung bei der therapeutischen Behandlung des menschlichen oder tierischen Körpers.

Dabei werden die erfindungsgemäßen Substanzen in der Regel in Analogie zu bekannten, im Handel befindlichen Psychopharmaka (z. B. Imipramin) verabreicht, vorzugsweise in Dosierungen zwischen etwa 2 und 500 mg, insbesondere zwischen 10 und 50 mg pro Dosierungseinheit. Die tägliche Dosierung liegt vorzugsweise zwischen etwa 0,05 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Jede einzelne der in den folgenden Beispielen genannten Verbindungen der Formel I ist zur Herstellung von pharmazeutischen Zubereitungen besonders geeignet.

In den nachfolgenden Beispielen bedeutet »übliche Aufarbeitung«:

Man gibt, falls erforderlich, Natronlauge hinzu, extrahiert mit einem organischen Lösungsmittel wie Benzol, Chloroform oder Dichlormethan, trennt ab, trocknet die organische Phase über Natriumsulfat, filtriert, dampft ein und reinigt durch Chromatographie und/oder Kristallisation.

Die Rf-Werte wurden an Kieselgel mit dem jeweils angegebenen Lösungsmittelgemisch erhalten: (A) = Toluol/Triäthylamin 8 : 2, (B) = Methylenchlorid/Methanol 9 : 1.

## Beispiel 1

Man löst 16,2 g p-Trifluormethylphenol in 150 ml 0,5n äthanolischem KOH und rührt eine Stunde bei 20°. Es wird eingedampft und der Rückstand in 140 ml DMF aufgenommen. Man gibt eine Suspension von 23 g 1-Cyclopropyl-3-methylamino-propylbromid in 40 ml DMF hinzu, erwärmt das Gemisch 1,5 Stunden auf 130°, dampft erneut ein, arbeitet wie üblich auf und erhält 1-Methylamino-3-cyclopropyl-3-p-trifluormethylphenoxy-propan. Rf 0,22 (A).

## Beispiel 2

Man löst 16,2 g p-Trilfuormethylphenol in 500 ml absolutem Toluol, versetzt mit 7,1 ml Thallium-(I)-äthylat und rührt 1 Stunde bei 20°. Nach dem Eindampfen wird der Rückstand in 100 ml absolutem Acetonitril gelöst, mit 28,3 g 1-Cyclopropyl-3-methylaminopropyl-p-toluolsulfonat versetzt, 3 Stunden unter Rühren gekocht und eingedampft. Nach üblicher Aufarbeitung erhält man 1-Methylamino-3-cyclopropyl-3-trifluormethylphenoxy-propan. Rf 0,22 (A).

## Beispiel 3

Man löst 2,7 g Azodicarbonsäurediäthylester in 25 ml THF, gibt unter Kühlung und Rühren 4 g Triphenylphosphin hinzu, tropft anschließend eine Lösung von 2,1 g 1-Cyclopropyl-3-methyl-amino-propan-1-ol in 10 ml THF und dann eine Lösung von 1,4 g p-Trifluormethylphenol in 10 ml THF hinzu und rührt 2 Stunden bei 0°. Nach Stehenlassen über Nacht bei 20° wird wie üblich aufgearbeitet. Man erhält 1-Methyl-amino-3-cyclopropyl-3-p-trifluormethylphenoxy-propan. Rf 0,22 (A).

## Beispiel 4

Zu einer Lösung von 16,2 g p-Trifluormethylphenol in 100 ml $CH_2Cl_2$ gibt man 200 ml 50%ige wässerige Natronlauge und 1 g Triäthylbenzylammoniumchlorid, tropft unter Rühren 17,7 g 1-Cyclopropyl-3-methylamino-propylchlorid hinzu und rührt noch eine Stunde nach.

Nach üblicher Aufarbeitung erhält man 1-Methylamino-3-cyclo-propyl-3-p-trifluormethylphenoxy-propan. Rf 0,22 (A).

## Beispiel 5 bis 11

Analog Beispiel 1, 2, 3 oder 4 erhält man aus den entsprechenden Phenolen der Formel II und den entsprechenden Aminen der Formel III (z. B. 1-Cyclopropyl-3-methylamino-propan-1-ol, -propylchlorid, -propylbromid oder -propyljodid, 1-Cyclopropyl-3-dimethylamino-propan-1-ol, -propylchlorid, -propylbromid oder -propyljodid, 1-Cyclopropyl-3-N-benzyl-N-methylamino-propan-1-ol, -propylchlorid, -propylbromid oder -propyljodid):

5. 1-Methylamino-3-cyclopropyl-3-phenoxy-propan, Rf 0,4 (Dichlormethan/Triäthylamin 8 : 2).
6. 1-Methylamino-3-cyclopropyl-3-o-fluorphenoxy-propan.

7. 1-Methylamino-3-cyclopropyl-3-m-fluorphenoxy-propan.
8. 1-Methylamino-3-cyclopropyl-3-p-fluorphenoxy-propan, Hydrochlorid, F. 178–181°.
9. 1-Methylamino-3-cyclopropyl-3-o-chlorphenoxy-propan.
10. 1-Methylamino-3-cyclopropyl-3-m-chlorphenoxy-propan.
11. 1-Methylamino-3-cyclopropyl-3-p-chlorphenoxy-propan, Rf 0,4 (Dichlormethan/Triäthylamin 8 : 2).
12. 1-Methylamino-3-cyclopropyl-3-p-bromphenoxy-propan.
13. 1-Methylamino-3-cyclopropyl-3-o-tolyloxy-propan.
14. 1-Methylamino-3-cyclopropyl-3-m-tolyloxy-propan.
15. 1-Methylamino-3-cyclopropyl-3-p-tolyloxy-propan, Rf 0,3 (Dichlormethan/Triäthylamin 8 : 2).
16. 1-Methylamino-3-cyclopropyl-3-p-n-butylphenoxy-propan.
17. 1-Methylamino-3-cyclopropyl-3-o-methoxyphenoxy-propan, Rf 0,3 (Dichlormethan/Triäthylamin 8 : 2).
18. 1-Methylamino-3-cyclopropyl-3-m-methoxyphenoxy-propan, Rf 0,4 (Chloroform/Triäthylamin 8 : 2).
19. 1-Methylamino-3-cyclopropyl-3-p-methoxyphenoxy-propan, Rf 0,43 (Chloroform/Triäthylamin 8 : 2).
20. 1-Methylamino-3-cyclopropyl-3-p-n-butoxyphenoxy-propan.
21. 1-Methylamino-3-cyclopropyl-3-o-allyloxyphenoxy-propan, Rf 0,58 (Chloroform/Triäthylamin 8 : 2).
22. 1-Methylamino-3-cyclopropyl-3-m-allyloxyphenoxy-propan.
23. 1-Methylamino-3-cyclopropyl-3-p-allyloxyphenoxy-propan.
24. 1-Methylamino-3-cyclopropyl-3-p-(2-methyl-allyloxy)-phenoxy-propan.
25. 1-Methylamino-3-cyclopropyl-3-o-methylthiophenoxy-propan.
26. 1-Methylamino-3-cyclopropyl-3-m-methylthiophenoxy-propan.
27. 1-Methylamino-3-cyclopropyl-3-p-methylthiophenoxy-propan, Rf 0,60 (Chloroform/Triäthylamin 8 : 2).
28. 1-Methylamino-3-cyclopropyl-3-p-n-butylthiophenoxy-propan.
29. 1-Methylamino-3-cyclopropyl-3-o-hydroxyphenoxy-propan.
30. 1-Methylamino-3-cyclopropyl-3-m-hydroxyphenoxy-propan.
31. 1-Methylamino-3-cyclopropyl-3-p-hydroxyphenoxy-propan.
32. 1-Methylamino-3-cyclopropyl-3-o-mercaptophenoxy-propan.
33. 1-Methylamino-3-cyclopropyl-3-m-mercaptophenoxy-propan.
34. 1-Methylamino-3-cyclopropyl-3-p-mercaptophenoxy-propan.
35. 1-Methylamino-3-cyclopropyl-3-o-cyanphenoxy-propan.
36. 1-Methylamino-3-cyclopropyl-3-m-cyanphenoxy-propan.
37. 1-Methylamino-3-cyclopropyl-3-cyanphenoxy-propan, Rf 0,3 (Chloroform/Triäthylamin 8 : 2).
38. 1-Methylamino-3-cyclopropyl-3-(3,4-dimethoxy-phenoxy)-propan, Rf 0,35 (Chloroform).
39. 1-Methylamino-3-cyclopropyl-3-(3,4-methylendioxy-phenoxy)-propan, Rf 0,45 (Chloroform/Triäthylamin 8 : 2).
40. 1-Methylamino-3-cyclopropyl-3-o-trifluormethylphenoxy-propan.
41. 1-Methylamino-3-cyclopropyl-3-m-trifluormethylphenoxy-propan.
42. 1-Dimethylamino-3-cyclopropyl-3-phenoxy-propan, Rf 0,75 (Chloroform/Triäthylamin 8 : 2).
43. 1-Dimethylamino-3-cyclopropyl-3-o-fluorphenoxy-propan.
44. 1-Dimethylamino-3-cyclopropyl-3-m-fluorphenoxy-propan, Rf 0,75 (Chloroform/Triäthylamin 8 : 2).
45. 1-Dimethylamino-3-cyclopropyl-3-p-fluorphenoxy-propan.
46. 1-Dimethylamino-3-cyclopropyl-3-o-chlorphenoxy-propan.
47. 1-Dimethylamino-3-cyclopropyl-3-m-chlorphenoxy-propan.
48. 1-Dimethylamino-3-cyclopropyl-3-p-chlorphenoxy-propan, Rf 0,5 (Aceton/Triäthylamin 9 : 1).
49. 1-Dimethylamino-3-cyclopropyl-3-p-bromphenoxy-propan.
50. 1-Dimethylamino-3-cyclopropyl-3-o-tolyloxy-propan.
51. 1-Dimethylamino-3-cyclopropyl-3-m-tolyloxy-propan.
52. 1-Dimethylamino-3-cyclopropyl-3-p-tolyloxy-propan, Rf 0,46 (Toluol/Triäthylamin 9 : 1).
53. 1-Dimethylamino-3-cyclopropyl-3-o-methoxyphenoxy-propan, Rf 0,25 (A).
54. 1-Dimethylamino-3-cyclopropyl-3-m-methoxyphenoxy-propan, Rf 0,7 (Chloroform/Triäthylamin 8 : 2).
55. 1-Dimethylamino-3-cyclopropyl-3-p-methoxyphenoxy-propan, Rf 0,41 (Toluol/Triäthylamin 9 : 1).
56. 1-Dimethylamino-3-cyclopropyl-3-o-allyloxyphenoxy-propan, Rf 0,48 (Toluol/Triäthylamin 9 : 1).
57. 1-Dimethylamino-3-cyclopropyl-3-m-allyloxyphenoxy-propan.
58. 1-Dimethylamino-3-cyclopropyl-3-p-allyloxyphenoxy-propan.
59. 1-Dimethylamino-3-cyclopropyl-3-o-methylthiophenoxy-propan.
60. 1-Dimethylamino-3-cyclopropyl-3-m-methylthiophenoxy-propan.
61. 1-Dimethylamino-3-cyclopropyl-3-p-methylthiophenoxy-propan, Rf 0,45 (Toluol/Triäthylamin 9 : 1).

62. 1-Dimethylamino-3-cyclopropyl-3-o-hydroxyphenoxy-propan.
63. 1-Dimethylamino-3-cyclopropyl-3-m-hydroxyphenoxy-propan.
64. 1-Dimethylamino-3-cyclopropyl-3-p-hydroxyphenoxy-propan.
65. 1-Dimethylamino-3-cyclopropyl-3-o-mercaptophenoxy-propan.
66. 1-Dimethylamino-3-cyclopropyl-3-m-mercaptophenoxy-propan.
67. 1-Dimethylamino-3-cyclopropyl-3-p-mercaptophenoxy-propan.
68. 1-Dimethylamino-3-cyclopropyl-3-o-cyanphenoxy-propan.
69. 1-Dimethylamino-3-cyclopropyl-3-m-cyanphenoxy-propan.
70. 1-Dimethylamino-3-cyclopropyl-3-p-cyanphenoxy-propan.
71. 1-Dimethylamino-3-cyclopropyl-3-(3,4-dimethoxyphenoxy)-propan.
72. 1-Dimethylamino-3-cyclopropyl-3-(3,4-methylendioxyphenoxy)-propan, Rf 0,42 (Toluol/Triäthylamin 9 : 1).
73. 1-Dimethylamino-3-cyclopropyl-3-o-trifluormethylphenoxy-propan.
74. 1-Dimethylamino-3-cyclopropyl-3-m-trifluormethylphenoxy-propan.
75. 1-Dimethylamino-3-cyclopropyl-3-p-trifluormethylphenoxy-propan, Rf 0,44 (Toluol/Triäthylamin 9 : 1), Hydrochlorid, F. 131 – 132°.
76. 1-N-Benzyl-N-methylamino-3-cyclopropyl-3-phenoxy-propan.
77. 1-N-Benzyl-N-methylamino-3-cyclopropyl-3-o-fluorphenoxy-propan.
78. 1-N-Benzyl-N-methylamino-3-cyclopropyl-3-m-fluorphenoxy-propan.
79. 1-N-Benzyl-N-methylamino-3-cyclopropyl-3-p-fluorphenoxy-propan, Rf 0,53 (B).
80. 1-N-Benzyl-N-methylamino-3-cyclopropyl-3-o-chlorphenoxy-propan.
81. 1-N-Benzyl-N-methylamino-3-cyclopropyl-3-m-chlorphenoxy-propan.
82. 1-N-Benzyl-N-methylamino-3-cyclopropyl-3-p-chlorphenoxy-propan.
83. 1-N-Benzyl-N-methylamino-3-cyclopropyl-3-p-bromphenoxy-propan.
84. 1-N-Benzyl-N-methylamino-3-cyclopropyl-3-o-tolyloxy-propan.
85. 1-N-Benzyl-N-methylamino-3-cyclopropyl-3-m-tolyloxy-propan.
86. 1-N-Benzyl-N-methylamino-3-cyclopropyl-3-p-tolyloxy-propan.
87. 1-N-Benzyl-N-methylamino-3-cyclopropyl-3-o-methoxyphenoxy-propan.
88. 1-N-Benzyl-N-methylamino-3-cyclopropyl-3-m-methoxyphenoxy-propan.
89. 1-N-Benzyl-N-methylamino-3-cyclopropyl-3-p-methoxyphenoxy-propan, Rf 0,81 (B).
90. 1-N-Benzyl-N-methylamino-3-cyclopropyl-3-o-allyloxyphenoxy-propan.
91. 1-N-Benzyl-N-methylamino-3-cyclopropyl-3-m-allyloxyphenoxy-propan.
92. 1-N-Benzyl-N-methylamino-3-cyclopropyl-3-p-allyloxyphenoxy-propan.
93. 1-N-Benzyl-N-methylamino-3-cyclopropyl-3-o-methylthiophenoxy-propan.
94. 1-N-Benzyl-N-methylamino-3-cyclopropyl-3-m-methylthiophenoxy-propan.
95. 1-N-Benzyl-N-methylamino-3-cyclopropyl-3-p-methylthiophenoxy-propan, Rf 0,65 (B).
96. 1-N-Benzyl-N-methylamino-3-cyclopropyl-3-o-hydroxyphenoxy-propan.
97. 1-N-Benzyl-N-methylamino-3-cyclopropyl-3-m-hydroxyphenoxy-propan.
98. 1-N-Benzyl-N-methylamino-3-cyclopropyl-3-p-hydroxyphenoxy-propan.
99. 1-N-Benzyl-N-methylamino-3-cyclopropyl-3-o-mercaptophenoxy-propan.
100. 1-N-Benzyl-N-methylamino-3-cyclopropyl-3-m-mercaptophenoxy-propan.
101. 1-N-Benzyl-N-methylamino-3-cyclopropyl-3-p-mercaptophenoxy-propan.
102. 1-N-Benzyl-N-methylamino-3-cyclopropyl-3-o-cyanphenoxy-propan.
103. 1-N-Benzyl-N-methylamino-3-cyclopropyl-3-m-cyanphenoxy-propan.
104. 1-N-Benzyl-N-methylamino-3-cyclopropyl-3-p-cyanphenoxy-propan.
105. 1-N-Benzyl-N-methylamino-3-cyclopropyl-3-(3,4-dimethoxyphenoxy)-propan.
106. 1-N-Benzyl-N-methylamino-3-cyclopropyl-3-(3,4-methylen-dioxyphenoxy)-propan.
107. 1-N-Benzyl-N-methylamino-3-cyclopropyl-3-o-trifluormethylphenoxy-propan.
108. 1-N-Benzyl-N-methylamino-3-cyclopropyl-3-m-trifluormethylphenoxy-propan.
109. 1-N-Benzyl-N-methylamino-3-cyclopropyl-3-p-trifluormethylphenoxy-propan.
110. 1-N-Äthyl-N-methylamino-3-cyclopropyl-3-p-trifluormethylphenoxy-propan.
111. 1-N-n-Butyl-N-methylamino-3-cyclopropyl-3-p-trifluormethylphenoxy-propan.

## Beispiel 112

Eine Lösung von 4,14 g 1-p-Toluolsulfonyloxy-3-cyclopropyl-3-p-trifluormethylphenoxy-propan (erhältlich durch Reaktion von Cyclopropylcyanid mit 2,2-Diäthoxyäthylmagnesiumbromid und anschließende Hydrolyse zu 3,3-Diäthoxy-1-cyclopropyl-propan-1-on, Reduktion zu 3,3-Diäthoxy-1-cyclopropyl-propan-1-ol, Umsetzung mit p-Trifluormethylphenol analog Beispiel 3, Hydrolyse zu 3-Cyclopropyl-3-p-trifluormethylphenoxy-propanal, Reduktion zu 3-Cyclopropyl-3-p-trifluormethylphenoxy-propan-1-ol und Tosylierung) 30 g Methylamin in 100 ml Methanol wird im Autoklaven 2 Stunden auf 120° erhitzt. Nach dem Abkühlen und üblicher Aufarbeitung erhält man 1-Methylamino-3-cyclopropyl-3-p-trifluormethylphenoxy-propan. Rf 0,22 (A).

## Beispiele 113 bis 219

Analog Beispiel 112 erhält man durch Umsetzung der entsprechenden 1-p-Toluolsulfonyloxy-, 1-Chlor- oder 1-Brom-3-cyclopropyl-3-aryloxy-propane mit Methylamin, Dimethylamin, N-Benzyl-N-methylamin, N-Äthyl-N-methylamin oder N-n-Butyl-N-methylamin die in den Beispielen 5 bis 111 angegebenen Verbindungen. Dabei kann man mit den weniger flüchtigen Aminen auch auf die Anwendung von Druck verzichten und/oder höhersiedende Lösungsmittel wie Isopropanol oder n-Butanol verwenden.

## Beispiel 220

Man löst 2,3 g Natrium in 250 ml Äthanol, versetzt mit 24,2 g 3-Cyclopropyl-3-p-trifluormethylphenoxy-propen (erhältlich aus 3-Cyclopropyl-3-hydroxypropen und p-Trifluormethylphenol), dann mit einer Lösung von 100 g Methylamin in 300 ml Äthanol und erhitzt das Gemisch im Autoklaven 72 Stunden auf 100°. Nach Eindampfen und üblicher Aufarbeitung erhält man 1-Methylamino-3-cyclopropyl-3-p-trifluormethylphenoxy-propan. Rf 0,22 (A).

## Beispiele 221 bis 327

Analog Beispiel 220 erhält man durch Anlagerung der obengenannten Amine an die entsprechenden 3-Cyclopropyl-3-aryloxy-propene die in den Beispielen 5 bis 111 angegebenen Verbindungen.

## Beispiel 328

Eine Lösung von 2,59 g 1-Amino-3-cyclopropyl-3-p-trifluormethylphenoxy-propan (erhältlich durch Kondensation von Cyclopropancarbonsäureäthylester mit Äthylacetat zu 3-Cyclopropyl-propan-3-on-1-säureäthylester, Reduktion zu 3-Cyclopropyl-propan-3-ol-1-säureäthylester, Reaktion mit p-Trifluormethylphenol analog Beispiel 3 zu 3-Cyclopropyl-3-p-trifluormethylphenoxy-propansäureäthylester, Reaktion mit $NH_3$ zum Amid und Reduktion mit $LiAlH_4$) und 1,5 g Benzaldehyd in 25 ml Toluol wird eine Stunde am Wasserabscheider gekocht. Die Lösung des erhaltenen 1-Benzylidenamino-3-cyclopropyl-3-p-trifluormethylphenoxy-propans wird mit 5 g Methyljodid 12 Stunden auf 150° im Rohr erhitzt und danach eingedampft. Das erhaltene quartäre Salz wird 10 Minuten in 90%igem Äthanol gekocht. Man dampft erneut ein, nimmt in verdünnter Salzsäure auf und entfernt den abgespaltenen Benzaldehyd durch Extraktion mit Äther. Die saure wässerige Lösung wird mit Natronlauge alkalisch gemacht und wie üblich aufgearbeitet. Man erhält 1-Methylamino-3-cyclopropyl-3-p-trifluormethylphenoxy-propan. Rf 0,22 (A).

## Beispiele 329 bis 365

Analog Beispiel 328 erhält man durch Methylierung der entsprechenden 1-Amino-3-cyclopropyl-3-aryloxy-propane die in den Beispielen 5 bis 41 angegebenen Verbindungen.

## Beispiel 366

Ein Gemisch aus 29,6 g 1-Amino-3-cyclopropyl-3-trifluormethylphenoxy-propan-hydrochlorid, 50 ml Ameisensäure, 7 g Natriumformiat und 40 ml 40%iger Formaldehyd-Lösung wird 3 Stunden auf 60° und danach 12 Stunden auf 100° erhitzt. Nach üblicher Aufarbeitung erhält man 1-Dimethylamino-3-cyclopropyl-3-p-trifluormethylphenoxy-propan.

## Beispiele 367 bis 400

Analog Beispiel 366 erhält man aus den entsprechenden 1-Amino-3-cyclopropyl-3-aryloxy-propanen mit Ameisensäure/Natriumformiat/Formaldehyd die in den Beispielen 42 bis 75 beschriebenen Verbindungen.

## Beispiel 401

Ein Gemisch aus 29,8 g 1-N-Methyl-N-cyan-amino-3-cyclopropyl-3-trifluormethylphenoxy-propan (erhältlich durch Reaktion von 1-Dimethylamino-3-cyclopropyl-3-p-trifluormethylphenoxy-propan mit

BrCN), 300 g KOH, 250 ml Wasser und 1200 ml Äthylenglykol wird 20 Stunden gekocht, abgekühlt und wie üblich aufgearbeitet. Man erhält 1-Methylamino-3-cyclopropyl-3-p-trifluormethylphenoxy-propan. Rf 0,22 (A).

Beispiele 402 bis 438

Analog Beispiel 401 erhält man durch Reaktion der entsprechenden 1-N-Methyl-N-ncyan-amino-3-cyclopropyl-3-aryloxy-propane mit KOH die in den Beispielen 5 bis 41 angegebenen Verbindungen.

Beispiel 439

Eine Lösung von 3,69 g 1-N-Methyl-N-trifluoracetylamino-3-cyclopropyl-3-p-trifluormethylphenoxy-propan (erhältlich durch Reaktion von 1-p-Toluolsulfonyloxy-3-cyclopropyl-3-p-trifluormethyl-phenoxy-propan mit N-Methyl-trifluoracetamid) in 50 ml 1n äthanolischer KOH-Lösung wird eine Stunde gekocht. Man dampft ein, arbeitet wie üblich auf und erhält 1-Methylamino-3-cyclopropyl-3-p-trifluormethylphenoxy-propan. Rf 0,22 (A).

Beispiele 440 bis 476

Analog Beispiel 439 erhält man aus entsprechenden 1-N-Methyl-N-acylamino-3-cyclopropyl-3-aryloxy-propanen durch alkalische Hydrolyse die in den Beispielen 5 bis 41 angegebenen Verbindungen.

Beispiel 477

Zu einer Suspension von 7,6 g $LiAlH_4$ in 250 ml absolutem THF tropft man unter Rühren eine Lösung von 28,7 g 3-Cyclopropyl-3-p-trifluormethylphenoxy-propansäure-N-methylamid (erhältlich durch Umsetzen des entsprechenden Äthylesters mit Methylamin) in 500 ml THF, kocht 16 Stunden, versetzt unter Kühlung mit Äthylacetat, dann mit 32%iger Natronlauge, arbeitet wie üblich auf und erhält 1-Methylamino-3-cyclopropyl-3-p-trifluormethyl-phenoxy-propan. Rf 0,22 (A).

Beispiele 478 bis 584

Analog Beispiel 477 erhält man durch Reduktion der entsprechenden 3-Cyclopropyl-3-aryloxy-propansäure-N-methylamide, -N,N-dimethylamide, -N-benzyl-N-methylamide, -N-äthyl-N-methylamide oder -N-n-butyl-N-methylamide die in Beispiel 5 bis 111 angegebenen Verbindungen.

Beispiel 585

Eine Lösung von 27,1 g 1-Methylimino-3-cyclopropyl-3-trifluormethylphenoxy-propan (erhältlich aus 3-Cyclopropyl-3-p-trifluormethylphenoxy-propanal und Methylamin) in 250 ml Dioxan wird an 2 g 2%igem Pd—C bei 20° und Normaldruck bis zur Aufnahme von 1 Mol Wasserstoff hydriert. Man filtriert, dampft ein und erhält 1-Methylamino-3-cyclopropyl-3-p-trifluormethylphenoxy-propan. Rf 0,22 (A).

Beispiele 586 bis 622

Analog Beispiel 585 erhält man durch Hydrierung der entsprechenden 1-Methylimino-3-cyclopropyl-3-aryloxy-propane die in den Beispielen 5 bis 41 angegebenen Verbindungen.

Beispiel 623

Zu 27,3 g 1-Methylamino-3-cyclopropyl-3-p-trifluormethylphenoxy-propan tropft man unter Rühren und Kühlen 30 g Ameisensäure und anschließend bei 20° 7 g 25%ige Formaldehydlösung. Man erhitzt bis zum Ende der Gasentwicklung auf dem Wasserbad, kühlt ab, gießt auf Eis, arbeitet wie üblich auf und erhält 1-Dimethylamino-3-cyclopropyl-3-p-trifluormethylphenoxy-propan.

Beispiel 624

Man gibt 17,1 g Benzylbromid und 26 g wasserfreies Kaliumcarbonat zu einer Lösung von 27,3 g 1-Methylamino-3-cyclopropyl-3-p-trifluormethylphenoxy-propan in 1 l absolutem Toluol, kocht 20 Stunden, kühlt ab, gießt in Wasser, arbeitet wie üblich auf und erhält 1-N-Benzyl-N-methylamino-3-cyclopropyl-3-p-trifluormethylphenoxy-propan.

Beispiel 625

Eine Lösung von 3,63 g 1-N-Benzyl-N-methylamino-3-cyclopropyl-3-p-trifluormethylphenoxy-propan in 70 ml Toluol wird mit 0,4 g $K_2CO_3$ versetzt. Unter Rühren und Sieden tropft man 5,9 g Chlorameisensäure-2,2,2-trichloräthylester hinzu, kocht noch 3 Stunden, gießt auf Eis, trennt ab, trocknet die organische Phase über $MgSO_4$ und dampft ein. Das erhaltene rohe 1-N-Methyl-N-(2,2,2-trichloräthoxy-carbonyl)-amino-3-cyclopropyl-3-p-trifluormethylphenoxy-propan (4,7 g) wird in 50 ml 95%ige Essigsäure eingetragen. Man gibt 8,5 g Zinkpulver hinzu, rührt 3 Stunden bei 25°, filtriert, arbeitet das Filtrat wie üblich auf und erhält 1-Methylamino-3-cyclopropyl-3-p-trifluormethyl-phenoxy-propan. Rf 0,22 (A).

Beispiel 626

Eine Lösung von 36,3 g 1-N-Benzyl-N-methylamino-3-cyclopropyl-3-p-trifluormethyl-phenoxy-propan in 400 ml Methanol wird an 3 g 5%igem Pd—C bei 20° und 1 at bis zur Aufnahme von 0,1 Mol Wasserstoff hydriert. Man filtriert, dampft ein und erhält 1-Methylamino-3-cyclopropyl-3-p-trifluormethyl-phenoxy-propan. Rf 0,22 (A).

Beispiel 627

Ein Gemisch aus 1 g 1-Dimethylamino-3-cyclopropyl-3-p-methoxyphenoxy-propan und 1 g Pyridinhydrochlorid wird 3 Stunden bei 160° gerührt. Nach üblicher Aufarbeitung erhält man 1-Dimethylamino-3-cyclopropyl-3-p-hydroxyphenoxy-propan.

Beispiel 628

Analog Beispiel 627 erhält man aus 1-Dimethylamino-3-cyclopropyl-3-p-methylthiophenoxy-propan mit Pyridinhydrochlorid das 1-Dimethylamino-3-cyclopropyl-3-p-mercaptophenoxy-propan.

Beispiel 629

Ein Gemisch aus 23,5 g 1-Dimethylamino-3-cyclopropyl-3-p-hydroxyphenoxy-propan, 100 ml absolutes Äthanol und 200 ml 0,5n äthanolisches KOH wird eine Stunde bei 20° gerührt und eingedampft. Man löst den Rückstand in 250 ml absolutem DMF und versetzt portionsweise unter Rühren mit 12,6 g Dimethylsulfat. Man kocht das Gemisch zwei Stunden, dampft ein, arbeitet wie üblich auf und erhält 1-Dimethylamino-3-cyclopropyl-3-p-methoxyphenoxy-propan.

Beispiel 630

Analog Beispiel 629 erhält man aus 1-Dimethylamino-3-cyclopropyl-3-p-mercaptophenoxy-propan mit Dimethylsulfat das 1-Dimethylamino-3-cyclopropyl-3-p-methylthiophenoxy-propan.

Die nachstehenden Beispiele betreffen pharmazeutische Zubereitungen, die Amine der Formel I oder ihre Säureadditionssalze enthalten.

Beispiel A

Tabletten

Ein Gemisch von 1 kg 1-Methylamino-3-cyclopropyl-3-p-trifluormethyl-phenoxy-propan-hydrochlorid, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B

Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C

Kapseln

2 kg 1-Methylamino-3-cyclopropyl-3-p-trifluormethylphenoxy-propan-hydrochlorid werden in üblicher Weise in Hartgelatinekapseln gefüllt, so daß jede Kapsel 20 mg des Wirkstoffes enthält.

Beispiel D

Ampullen

Eine Lösung von 1 kg 1-Methylamino-3-cyclopropyl-3-p-trifluormethyl-phenoxy-propan-hydrochlorid in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Patentansprüche**

1. Cyclopropanderivate der allgemeinen Formel I

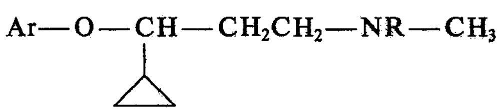

I

worin

Ar Phenyl oder eine ein- oder mehrfach durch F, Cl, Br, Alkyl, Alkoxy, Alkenyloxy und/oder Alkylthio mit jeweils bis zu 4 C-Atomen, OH, SH, CN, Methylendioxy und/oder $CF_3$ substituierte Phenylgruppe und

R H, Alkyl mit 1–4 C-Atomen oder Benzyl

bedeuten, sowie deren physiologisch unbedenkliche Säureadditionssalze.

2. 1-Methylamino-3-cyclopropyl-3-p-trifluormethyl-phenoxypropan.

3. Verfahren zur Herstellung von Cyclopropanderivaten der allgemeinen Formel I

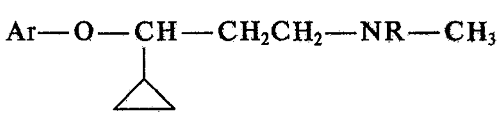

I

worin

Ar Phenyl oder eine ein- oder mehrfach durch F, Cl, Br, Alkyl, Alkoxy, Alkenyloxy und/oder Alkylthio mit jeweils bis zu 4 C-Atomen, OH, SH, CN, Methylendioxy und/oder $CF_3$ substituierte Phenylgruppe und

R H, Alkyl mit 1–4 C-Atomen oder Benzyl

bedeuten, sowie von deren physiologisch unbedenklichen Säureadditionssalzen, dadurch gekennzeichnet, daß man ein Phenol der allgemeinen Formel II

Ar–OH II

worin

Ar die angegebene Bedeutung hat,

oder eines seiner Salze mit einem Amin der allgemeinen Formel III

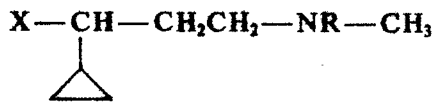 III

worin

X Cl, Br, J oder OH bedeutet und
R die angegebene Bedeutung hat,

oder mit einem seiner reaktionsfähigen Derivate umsetzt

oder daß man ein Cyclopropanderivat der allgemeinen Formel IV

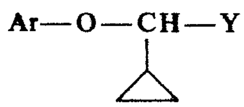 IV

worin

Y $-CH_2CH_2X$ oder $-CH=CH_2$ bedeutet und
Ar und X die angegebenen Bedeutungen haben,

oder eines seiner reaktionsfähigen Derivate mit einem Amin der allgemeinen Formel V

$HNR-CH_3$ V

worin

R die angegebene Bedeutung hat,

umsetzt

oder daß man ein Amin der allgemeinen Formel VI

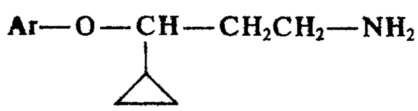 VI

worin

Ar die angegebene Bedeutung hat,

mit einem alkylierenden Mittel behandelt
oder daß man eine Verbindung, die der allgemeinen Formel I entspricht, jedoch an Stelle eines Wasserstoffatoms zusätzlich eine solvolytisch abspaltbare Gruppe enthält,

mit einem solvolysierenden Mittel behandelt
oder daß man eine Verbindung, die der allgemeinen Formel I entspricht, jedoch an Stelle von Wasserstoffatomen eine oder mehrere reduzierbare Gruppen und/oder eine oder mehrere C–C- und/oder C–N-Mehrfachbindungen enthält, mit einem reduzierenden Mittel behandelt und/oder daß man gegebenenfalls in einer Verbindung der Formel I eine sekundäre Aminogruppe durch Behandeln mit einem alkylierenden oder benzylierenden Mittel in die entsprechende tertiäre Aminogruppe und/oder eine Hydroxygruppe durch Behandeln mit einem alkylierenden oder alkenylierenden Mittel in die entsprechende Alkoxy- oder Alkenyloxygruppe und/oder eine Mercaptogruppe durch Behandeln mit einem alkylierenden Mittel in die entsprechende Alkylthiogruppe und/oder eine N-Benzylgruppe durch Behandeln mit einem entbenzylierenden Mittel (falls erwünscht, stufenweise) in eine NH-Gruppe umwandelt und/oder eine Alkoxy- und/oder Alkylthiogruppe unter Bildung einer Hydroxy- und/oder Mercaptogruppe spaltet und/oder eine erhaltene Base der Formel I durch Behandeln mit einer Säure in eines ihrer physiologisch unbedenklichen Säureadditionssalze umwandelt.

4. Verfahren zur Herstellung pharmazeutischer Zubereitungen, dadurch gekennzeichnet, daß man

eine Verbindung der Formel I und/oder eines ihrer physiologisch unbedenklichen Säureadditionssalze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Säureadditionssalze.

**Claims**

1. Cyclopropane derivatives of the general formula

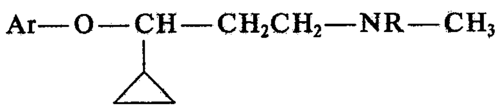
I

in which Ar is phenyl or a phenyl group which is monosubstituted or polysubstituted by F, Cl, Br, alkyl, alkoxy, alkenyloxy and/or alkylthio, having, in each case, not more than 4 C atoms, OH, SH, CN, methylenedioxy and/or $CF_3$ and R is H, alkyl having 1–4 C atoms or benzyl, and their physiologically acceptable acid addition salts.

2. 1-Methylamino-3-cyclopropyl-3-p-trifluoromethyl-phenoxypropane.

3. Process for the preparation of cyclopropane derivatives of the general formula I

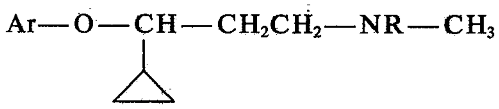
I

in which Ar is phenyl or a phenyl group which is monosubstituted or polysubstituted by F, Cl, Br, alkyl, alkoxy, alkenyloxy and/or alkylthio, having, in each case, not more than 4 C atoms, OH, SH, CN, methylenedioxy and/or $CF_3$ and R is H, alkyl having 1–4 C atoms or benzyl, and of their physiologically acceptable acid addition salts, characterised in that a phenol of the general formula II

Ar–OH II

in which Ar is as defined, or one of its salts, is reacted with an amine of the general formula III

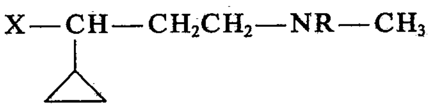
III

in which X is Cl, Br, I or OH and R is as defined, or with one of its reactive derivatives, or in that a cyclopropane derivative of the general formula IV

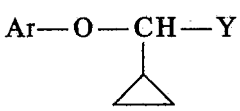
IV

in which Y is $-CH_2CH_2X$ or $-CH=CH_2$ and Ar and X are as defined, or one of its reactive derivatives, is reacted with an amine of the general formula V

$HNR-CH_3$ V

in which R is as defined, or in that an amine of the general formula VI

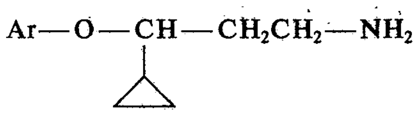
VI

in which Ar is as defined, is treated with an alkylating agent, or in that a compound which corresponds to the general formula I but additionally contains a solvolytically detachable group in place of a hydrogen atom is treated with a solvolysing agent, or in that a compound which corresponds to the general formula I but contains one or more reducible groups and/or one or more C–C and/or C–N multiple bonds in place of hydrogen atoms is treated with a reducing agent, and/or in that, optionally, in a compound of the formula I, a secondary amino group is converted by treatment with an alkylating

or benzylating agent into the corresponding tertiary amino group and/or a hydroxyl group is converted by treatment with an alkylating or alkenylating agent into the corresponding alkoxy or alkenyloxy group and/or a mercapto group is converted by treatment with an alkylating agent into the corresponding alkylthio group and/or a N-benzyl group is converted by treatment with a debenzylating agent (stepwise if desired) into a NH group and/or an alkoxy group and/or alkylthio group is split with the formation of a hydroxyl group and/or mercapto group and/or a resulting base of the formula I is converted by treatment with an acid into one of its physiologically acceptable acid addition salts.

4. Process for the preparation of pharmaceutical formulations, characterised in that a compound of the formula I and/or one of its physiologically acceptable acid addition salts is brought, together with at least one solid, liquid or semi-liquid excipient or auxiliary and, optionally, in combination with one or more further active compounds, into a suitable dosage form.

5. Pharmaceutical formulation, characterised in that it contains at least one compound of the general formula I and/or one of its physiologically acceptable acid addition salts.

**Revendications**

1. Dérivés de cyclopropane de formule générale I:

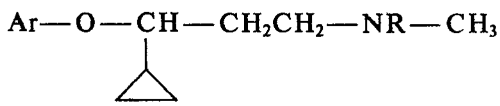

I

dans laquelle

Ar représente un groupe phényle ou un groupe phényle substitué une ou plusieurs fois par F, Cl, Br, un groupe alkyle, un groupe alcoxy, un groupe alcényloxy et/ou un groupe alkylthio contenant chacun jusqu'à 4 atomes de carbone, par un groupe OH, un groupe SH, un groupe CN, un groupe méthylène-dioxy et/ou par $CF_3$, et

R représente H, un groupe alkyle contenant 1 à 4 atomes de carbone ou un groupe benzyle,

ainsi que leurs sels d'addition d'acide physiologiquement acceptables.

2. Le 1-méthylamino-3-cyclopropyl-3-p-trifluorométhyl-phénoxypropane.

3. Procédé de préparation de dèrivés de cyclopropane de formule générale I:

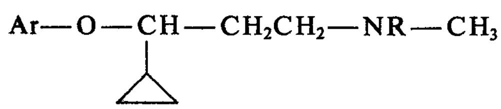

I

dans laquelle

Ar représente un groupe phényle ou un groupe phényle substitué une ou plusieurs fois par F, Cl, Br, un groupe alkyle, un groupe alcoxy, un groupe alcényloxy et/ou un groupe alkylthio contenant chacun jusqu'à 4 atomes de carbone, par un groupe OH, un groupe SH, un groupe CN, un groupe méthylène-dioxy et/ou par $CF_3$, et

R représente H, un groupe alkyle contenant 1 à 4 atomes de carbone ou un groupe benzyle,

ainsi que de leurs sels d'addition d'acide physiologiquement acceptables, caractérisé en ce qu'on fait réagir un phénol de formule générale II:

$$Ar-OH \qquad II$$

dans laquelle

Ar a la signification indiquée,

ou un de ses sels avec une amine de formule générale III:

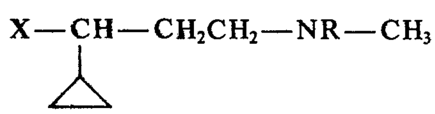

III

dans laquelle

X représente Cl, Br, I ou OH, et

R a la signification indiquée,

ou avec un de ses dérivés réactifs,

ou on fait réagir un dérivé de cyclopropane de formule générale IV:

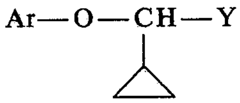

IV

dans laquelle

Y représente $-CH_2CH_2X$ ou $-CH=CH_2$, et
Ar et X ont les significations indiquées,

ou un de ses dérivés réactifs,

avec une amine de formule générale V:

$HNR-CH_3$ V

dans laquelle

R a la signification indiquée,

ou on traite une amine de formule générale VI:

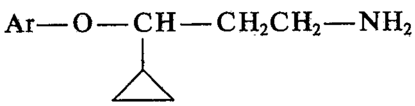

VI

dans laquelle

Ar a la signification indiquée,

avec un agent d'alkylation,
ou on traite un composé répondant à la formule générale I, mais contenant en plus, à la place d'un atome d'hydrogène, un groupe séparable par voie solvolytique,

avec un agent solvolysant,
ou on traite un composé répondant à la formule générale I, mais contenant, à la place d'atomes d'hydrogène, un ou plusieurs groupes réductibles et/ou une ou plusieurs liaisons multiples C–C et/ou C–N-, avec un agent réducteur et/ou, éventuellement dans un composé de formule I, on transforme un groupe amino secondaire en un groupe amino tertiaire correspondant par traitement avec un agent d'alkylation ou de benzylation et/ou on transforme un groupe hydroxy en un groupe alcoxy ou alcényloxy correspondant par traitement avec un agent d'alkylation ou d'alcénylation et/ou on transforme un groupe mercapto en un groupe alkylthio correspondant par traitement avec un agent d'alkylation et/ou dant par traitement avec un agent d'alkylation et/ou on transforme un groupe N-benzyle en un groupe NH par traitement avec un agent de débenzylation (si on le désire, par étapes) et/ou on sépare un groupe alcoxy et/ou un groupe alkylthio avec formation d'un groupe hydroxy et/ou d'un groupe mercapto et/ou on transforme une base obtenue de formule I en und de ses sels d'addition d'acide physiologiquement acceptables par traitement avec un acide.

4. Procédé en vue d'obtenir des préparations pharmaceutiques, caractérisé en ce qu'on met, sous une forme de dosage appropriée, un composé de formule I et/ou un de ses sels d'addition d'acide physiologiquement acceptables avec au moins une substance support ou une substance auxiliaire solide, liquide ou semi-liquide et éventuellement en combinaison avec une ou plusieurs autres substances actives.

5. Préparation pharmaceutique, caractérisée en ce qu'elle contient au moins un composé de formule générale I et/ou un de ses sels d'addition d'acide physiologiquement acceptables.